# EUROPEAN PATENT SPECIFICATION

(11) **EP 1 346 640 B1**
(45) Date of publication and mention of the grant of the patent: **09.05.2007**
(21) Application number: 02006228.7
(22) Date of filing: 20.03.2002
(51) Int. Cl.: A23G 1/00

(54) **A low fat cocoa extract**
Kakaoextrakt mit niedrigem Fettgehalt
Extrait de cacao à faible teneur en graisses

(43) Date of publication of application: 24.09.2003
(73) Proprietor: Société des Produits Nestlé S.A., 1800 Vevey (CH)
(72) Inventor: Mc Carthy, James,, 37210 Noizay (FR); Brevard, Hugues, 1805 Jongny (CH)
(74) Representative: Thomas, Alain

(56) References cited:
- EP-A- 0 749 694
- WO-A-01/93690
- GB-A- 693 666
- GB-A- 2 059 243
- US-A- 5 676 993
- US-B1- 6 312 753
- BIEHL B ET AL: "ACIDIFICATION, PROTEOLYSIS AND FLAVOUR POTENTIAL IN FERMENTING COCOA BEANS" JOURNAL OF THE SCIENCE OF FOOD AND AGRICULTURE, ELSEVIER APPLIED SCIENCE PUBLISHERS. BARKING, GB, vol. 7, no. 36, 1985, pages 583-598, XP001073935 ISSN: 0022-5142

## Description

### FIELD OF THE INVENTION

The present invention relates to a low fat cocoa extract with high flavor potential and to the process for its preparation.

### BACKGROUND OF THE INVENTION

Cocoa seeds have to be fermented after their harvest. Fermentation is a key operation in developing a first-rate flavor. If the fermentation process is not well conducted, the flavor will be weak in intensity and often spoiled by off-flavors; without fermentation, flavour characteristics can be present in small amounts but are generally unusable for industrial purposes. There are several fermentation systems, depending on the cacao variety and the country of origin. Usually, 200-400 kg of beans, with the pulp, are piled on banana leaves, covered with more leaves and mixed every second day for a week. Special crates are also used: the beans in the crates are mixed every two days by transfer from one to another. During the fermentation process, which usually lasts 3-6 days, several microorganisms develop naturally in the medium constituted by the heaps of beans and the surrounding pulp. During this period, the cocoa seeds lose their germinative power due to the breakdown of the cellular membranes, an event that also causes the cell contents to diffuse within the bean and thus become transformed enzymatically and chemically (Biehl, B. and Passern, D. (1982 J. Sci.Food Agric., 33, 1280-1290). These latter enzymatic and chemical changes include the production of cocoa flavor precursors and the reduction of negative attributes such as bitterness and astringency ("Industrial chocolate manufacture and use" by S.T. Beckett, third edition 1999, Blackwell Science, p17-19).

Several methods have been described in the past to alter the cocoa flavor properties of cocoa beans or cocoa liquor. For example, US 5888562 (equivalent to EP 7496949) to Nestec discloses an enzymatic treatment of cocoa for overcoming the variability in the flavor precursor composition. To achieve this goal, liquor from unfermented or underfermented cocoa beans is incubated for 2 hours at 50 °C in water whose pH is adjusted to 4.5 with a solution of acetic acid to activate endogenous cocoa endoproteinase. Then, the mixture is submitted to another enzymatic treatment which is necessary to obtain flavor precursors. More particularly, a nib or liquor is prepared from cocoa beans fermented for 1 to 15 days, it is mixed with an aqueous medium at pH 3-6, the mixture is incubated at 40-60°C for 10 minutes to 20 hours so as to promote the action of the cocoa endoprotease, the pH of the medium is adjusted to pH 4-8, at least one technical protease is added to it, and it is incubated at 10-60°C for 5 minutes to 20 hours.

WO 00/22935 to Kraft Jacobs Suchard discloses a low-flavor cocoa obtained from unfermented cocoa beans by a two-step process, the first step consisting of destroying the cellular and subcellular structures by treatment with an aqueous acetic acid solution and the second step consisting of an oxidation treatment. This method suppresses the formation of flavor and hence low-flavor cocoa is obtained which is useful as substitute for cocoa butter, for example.

Kirchhoff et al, in an article published in 1989 in "Food Chemistry" (Vol 31, pages 295-311), observed in a study of the "in- vitro fermentation" process that the acetic acid solution from the "in-vitro fermented" beans contained free amino acids, a significant proportion of which were hydrophobic amino acids.

GB-A-2 059 243 relates to a process for the fermentation of cocoa beans in order to produce fermented chocolate flavour precursors which are present in the fermented beans. The beans are then recovered and dried. Chocolates are prepared from the batch of beans fermented by the process described.

US-B-6 312 753 relates to the processing of cocoa beans or cocoa nibs which have been roasted. It also teaches that, in addition to the processed beans containing enhanced amounts of polyphenols, extracts may be prepared using solvents such as acetone, methanol, chloroform and ethyl acetate. The extracts are said to contain polyphenols but there is no mention of such extracts containing a high level of cocoa flavours and/or cocoa flavour precursors.

WO 01/93690 A discloses the extraction of defatted cocoa beans using methanol, ethanol, butanol, ethyl acetate or acetone as the solvent. The beans are treated before the extraction to remove the fat. The extract is said to contain polyphenols but there is no disclosure of an extract ontaining high levels of cocoa flavours and/or cocoa flavour precursors.

We have now found that, surprisingly, the acid solution (leachate) from "in-vitro-fermented" beans also contains a high level of cocoa flavor precursors, as well as high levels in cocoa antioxidants, such as polyphenols.

### SUMMARY OF THE INVENTION

We have developed acetic acid extracts generated during the "in vitro fermentation" of beans as a source of partially purified bio-active cocoa polyphenols and antioxidants, including leucocyanins, polyphenols, and the methylxanthines caffeine and theobromine, as well as a source of cocoa flavors and cocoa flavor precursors.

According to a first object of the invention, there is provided a low fat cocoa extract having a high level of cocoa flavor precursors and a high level of cocoa antioxidant compounds. According to a second aspect of the invention, there is provided a cocoa flavor concentrate and/or an antioxidant concentrate. The third aspect of the invention concerns the use of the extract as a source of antioxidants and/or cocoa flavor. According to the fourth aspect of the invention, there is provided a foodstuff enriched with the extract. The fifth aspect of the invention concerns the use of an acetic acid extract from an "in-vitro fermentation" to enhance cocoa flavor, especially at low levels, and the last aspect of the invention concerns a process for the preparation of the above-mentionned extract.

### DETAILED DESCRIPTION OF THE INVENTION

In the present description, by "dried acetic extract" and "extract", it is understood an acetic acid extract from the "in-vitro fermentation" concentrated to a paste or to a dry mass, and by "in-vitro fermentation" it is understood treatment of cocoa beans processed in acetic acid for more 4 hours. By "underfermented" cocoa beans or seeds it is understood beans or seeds fermented for about 1 to 4 days. Lastly, by cocoa "flavor" it is understood cocoa flavor and flavor precursors.

The present invention can use the process described below.
In the first part of the process according to this invention, fresh seeds or underfermented seeds are placed in 100 mM acetic acid in autoclave bottles, in such a way that the solution completely covers the beans in the bottles.
Then the bottles are placed in an incubator set at 40 to 55°C, preferably 50°C for a period of time comprised between 4 and 80 hours, or more preferably 12-72 hours, or even more preferably 12-60 hours, in a preferred embodiment for 12-48 hours.
When seeds are removed, the viscous incubation solutions generated are stored at - 20°C, and portions of this material are periodically thawed before the solids are concentrated by evaporation, for example by rotary evaporation under vacuum at 50°C. The solids obtained, hereby called the "in-vitro fermentation" extract are used in various analysis and experiments described below.
The seeds removed from the solution are placed in a hot air drier set at a temperature comprised between 50 and 70°C.

The extract obtained is a low fat cocoa extract (fat does not leach into the acetic acid solution), which surprisingly contains high levels of anti-oxidants and both cocoa flavors and cocoa flavor precursors. According to a first object of the invention, there is provided a low fat cocoa extract having a high level of cocoa flavor precursors and a high level of cocoa antioxidant compounds.
Cocoa flavor and cocoa flavor precursors containing amino groups were analysed, in the "in vitro fermented" solution, using the OPA method (Church F. et al, 1983, J. Dairy Science 66, 1219-1227) to determine the free amino nitrogen. Each of the acetic acid solutions are filtered and diluted, and the samples are reacted with 250 µL of the OPA solution. The mixes are allowed to react for 1 to 10 minutes, preferably 2 minutes, and then the absorbence is measured at 340 nm. The level of amino nitrogen containing molecules in the sample is calculated as leucine equivalents using an appropriate standard curve of different leucine concentrations reacted under the same conditions.

After a 48 hours incubation period, the "in-vitro fermentation" extract solution from beans of cacao variety CCN-51 contains from 2.74 to 3.1 mg/mL equivalents of leucine and the "in-vitro fermentation" extract solution from beans of the cacao variety EET-95 contains from 2.84 to 2.86 mg/mL equivalents of leucine. In the case of the CCN-51 solution, the solids content of the "in-vitro" fermented solution was determined to be 0.05 g/mL. Therefore, these results show that at least 5.6% of the solids in the CCN-51 acetic acid solution correspond to OPA reactive free amino nitrogen containing compounds such as the cocoa flavor precursors like aromatic amino acids and peptides.

SDS-PAGE gel analysis was performed to analyse the degradation of total cocoa bean proteins induced during the "in-vitro fermentation". In order to determine the level of protein degradation induced during the "in-vitro fermentation", acetone powders were made from these beans as described in Hansen et al. 1998 (J. Sci. Food Agric. 77, 273-281), after the pulp and testa have been removed. The total proteins in this extract were then analysed on a 10-20% Tris-Glycine SDS-Page gradient gel. The results showed that a significant amount of the bean proteins are degraded after 48 hours treatment. This is particularly clear for the two major vicilin proteins at approximately 48.5 and 34.1 kDa.
This analysis demonstrates that the acid incubation induced considerable protein degradation in the beans after 48 hours incubation, although it is less than that seen in a similar analysis of dried naturally fermented beans.

Total polyphenol content of the 48 hour acetic acid extract was analysed for the determination of polyphenol content. The spectrophotometric method (Folin-Ciocalteu Index) used to determine total polyphenol content closely corresponds to the method described in the Official Journal of European Communities, chapter 41 (3.10.1990, 178-179).

| | CCN-51 48 Hour Extract (dried solids) | CCN-51 Fresh Beans |
|---|---|---|
| Total Polyphenols | 32 mg ECE/g* | 26.3 mg ECE/g |

| | | |
|---|---|---|
| * mg ECE/g = milligrams of epicatechin equivalents per gram of sample | | |

The results obtained show that the 48 hours acetic acid extract of CCN-51 beans contains 32 mg ECE/g of dried solids. Thus, polyphenols represent 3.2% of the dried solids in the extract. Assuming that the fresh CCN-51 beans are approximately 45% solids, this implies the polyphenols represent 5.48% of the dried solids in CCN-51 beans. This information demonstrates that the ratio of polyphenols to total solids in the 48 hour acetic acid extract of CCN-51 beans is approximately half that seen for the dried solids in unfermented CCN-51 beans.

In order to characterize the compounds in the extracts further, these "in-vitro" fermentation solutions were extracted with 80%/20% acetone/water.
The 80%/20% acetone/water extracts of the acetic acid solutions obtained from the 48 hour "in-vitro fermented" EET-95 and CCN-51 beans, and the 80%/20% acetone/water extracts of fresh EET-95 and CCN-51 beans, were concentrated to dryness, solubilized in methanol and loaded on thin layer chromatography plates (TLC- 0.25 mm silica pre-coated plates; Merck, 60 - F₂₅₄). After development with solvent A (ethyl acetate 65%, methanol 23%, and water 12%) and reaction with dihydroxy-1,3-naphtalene (which detects reducing compounds) one strong diffuse spot was seen in the acetic acid extracts of both cocoa varieties. This intense diffuse spot corresponds primarily to the carbohydrates in the pulp because the fresh beans, which were stripped of their pulp and testa, had much lower levels of this spot. Development with solvent A and reaction with FeCL₃ (which detects phenolic compounds), indicated the presence of several similar spots in both the fresh beans and the concentrated acetic acid extract. This result shows that the phenolic composition of the acetic acid extracts are relatively similar to that seen of whole beans.
After development with solvent B (ethyl acetate 50%, methyl ethyl acetone 30%, formic acid 10%, and water 10%), a coloured spot was seen for both the "in-vitro fermentation" extracts and the control whole bean extracts. This spot, which is seen without any treatment of the TLC plates, is due to the seed anthocyanins. Examination of this TLC plate with UV illuminated a spot with the same migration as theobromine in both the fresh bean material and the "in-vitro fermentation" extracts. When this plate was subsequently reacted with ninhydrin, two relatively strong spots appeared in all the extracts, and these spots are probably due to the presence of amino acids and small peptides in the fresh beans and in the "in-vitro fermentation" extracts. Overall, the results indicate that the acetic acid extracts from the "in-vitro fermentation" have many of the same acetone/water soluble compounds present in the fresh seeds, and thus the "in-vitro fermentation" extracts probably have a significant proportion of the flavor and antioxidant molecules present in the cacao seeds.

As described above, the acetic acid extract contains amino acids and peptides, which are believed to be cocoa flavors and flavor precursors. To confirm the flavoring potential of these amino acids and peptides plus uncharacterised cocoa flavor precursors in the extracts, sniffing analysis of products formed after reacting the acetic acid extracts seeds of variety EET-95 and CCN-51 with fructose in a low water environment have been carried out. Before the reaction, the complete test mixes (extract, fructose, water, glycerol in ratio 1:1:1.5:96.5) had very little aroma and very little colour. However, after 1 hour of reaction, both test mixes had developed aromas with strong cocoa and caramel notes. Control experiments with lower fructose (0.1%) also had similar strong cocoa aromas. Other control reactions containing only 1% fructose, but with no acetic acid extract added, developed no cocoa aroma and had only a weak caramel aroma plus some clear off-notes. MS analysis of the products generated after a reaction of an acetic acid extract with sugar confirms that important cocoa aromas molecules, such as 2- and 3-methyl butanals, pentanedione, 2-methyl pyrazine, and phenylacetaldehyde are present in a heated reaction mix. Together, these results indicate that the 48 hour acetic acid extracts contain detectable cocoa aroma precursors.

In a preferred embodiment, the low fat cocoa extract contains flavors and/or flavor precursors which are in an amount of 2 to 6%, more preferably 6 to 12 %, and especially 20%. The content of polyphenols present in the extract can vary according to, for example, the maturation of the seed when harvested, the variety of cocoa or the degree of purification. Nevertheless, the polyphenol content of the extract as disclosed here can be from 10 to 32 mg epicatechin equivalents/g extract, more preferably 30 to 100 mg epicatechin equivalents/g extract, and in a preferred embodiment around 200 mg epicatechin equivalents/g extract.
Preferably, in this extract, the antioxidant compounds are bioactive.

According to a second aspect of the invention, there is provided a cocoa flavor concentrate or a cocoa flavor concentrate which has been reacted with sugars.
The cocoa flavor concentrate obtained by this reaction method can be used directly as produced. The said reaction allows at least part of the flavor precursors present in the concentrate to be transformed into cocoa flavors.
The sugars which can be used for this reaction are well known by the skilled person, and it is possible to use, among others, sucrose, fructose, glucose, molasses, starch degradation products (glucose or maltose syrups, glucose-fructose syrups, polydextrose), milk sugars, fruit sugars (including levulose), sorbitol, xylitol or manitol, glycerol or a mixture thereof. The glycerol used in the reaction could also be replaced by polyethylene glycol or related solutions and fats such as milk fat, cocoa butter or vegetable fats, for example.

In the second aspect of this invention, there is also provided an antioxidant concentrate obtainable by using an acetic acid extract from in-vitro fermentation. One way to obtain the antioxidant concentrate is to fractionate the original extract obtained from the in-vitro fermentation or to selectively purify it, by methods well known by those skilled in the art. This antioxidant concentrate can be used in any culinary products, as well as in the tires industry, the paintings industry, the iron industry, or in cosmetics and health products, among others.
The expression "culinary product" is intended to encompass any consumable matter. Hence, it may be a product intended for the consumption by humans, but the term also encompasses products to be consumed by animals, for example pets, such as dogs, cats, rabbits, guinea pigs, mice, rats, birds (for example parrots), reptiles and fish (for example goldfish). However, the term also includes food to be consumed by other domesticated animals, such as livestock, for example, cattle, horses, pigs, sheep, goats, buffaloes, camels, and the like.

The third aspect of the invention concerns the use of the dried extract as a source of antioxidants and/or cocoa flavor. As described above, the dried acetic acid extract contains polyphenols and anti-oxidants, including leucocyanins, polyphenols, the methylxanthines caffeine and theobromine. Thus, the extract, or fractions thereof, can be used, for example, in dietary supplements as an anti-oxidant source, or in any manufactured food as an anti-oxidant source; it can be used in replacement of chocolate, for example in breakfast cereals, yoghurts or other dairy products, cakes, biscuits, cereal bars, coatings, drinks and beverages, sweets, powder chocolates, baby foods, jelly products, ice creams, toppings and sauces. It can also be used in any culinary product as defined above.

According to the fourth aspect of the invention, there is provided a foodstuff enriched with the extract. Such foodstuff can be, for example, any culinary product as defined above, a dietary supplement, breakfast cereals, yoghurts or other dairy products, cakes, biscuits, cereal bars, coatings, drinks and beverages, sweets, powder chocolates, baby foods, jelly products, ice creams, toppings and sauces, and more generally any type of manufactured food.

The foodstuff can be enriched with the extract, or fractions thereof. The extract, or fractions thereof, added to said foodstuff can be the acetic acid extracts of fresh or underfermented cocoa beans, but it can also be the previously described extract which has been subjected to reactions with sugars. It can also be a mixture of these two extracts.
When the foodstuff is enriched with an extract which has been submitted to a reaction with sugars, it allows said foodstuff to develop a full cocoa flavor.

The fifth aspect of the invention concerns the use of an acetic acid extract from an "in-vitro fermentation" to enhance cocoa flavor. According to this aspect of the invention, the reacted dried acetic extract can be used in a preparation containing chocolate or chocolate extracts, as well as in preparations wherein the only chocolate source is the above-mentioned acetic acid extract, with or without sucrose, fructose, reducing sugars and/or other sweeteners.
The extract, reacted with sugars or not, can be added to the preparation at levels from 0.1% to 5.0%, when there is also another chocolate source, or at levels from 0.5% to 20% when there is no other chocolate source in the preparation.

Experiments have been performed to evaluate reacted dried acetic extracts to function as a cocoa flavor enhancer in a white chocolate model, at a level of 0.05% (final concentration). Various flavor reactions were carried out in two different low water environments at a concentration of 5% dried 48 hour CCN-51 acetic acid extract. The different low water reaction backgrounds used were either propylene glycol (PG) or anhydrous milk fat. Subsequently, the flavor potential of the reaction flavors generated were evaluated in white chocolate at a 1% incorporation level. One other test, in which 1% of the dried 48 hour CCN-51 unreacted extract was incorporated directly into the white chocolate, was also carried out.

| Medium | Ingredients | Temp (°C) | Time (min) | Incorporation in final chocolate product (%)* |
|---|---|---|---|---|
| 92%PG, 1.5% alkalized Water | 5% extract CCN51 + 1.5% Fructose | 125 | 60 | 1 § |
| 95% Anh. Milk fat | 5% extract CCN51 | 125 | 60 | 1 |
| No reaction | pure extract CCN51 lyoph. | | | 1 |

| | | | | |
|---|---|---|---|---|
| *O.3% lecithin was also added in this sample | | | | |

The products obtained were then tasted by an informal laboratory panel. The panel detected a weak cocoa flavor in the milk fat sample, and a weak cocoa like flavor in the sample with the 1% extract added.

### EXAMPLES

The following examples are illustrative of some of the products and methods of making the same falling within the scope of the present invention. They are not to be considered in any way limitative of the invention. Changes and modifications can be made with respect to the invention. That is, the skilled person will recognise many variations in these examples to cover a wide range of formulas, ingredients, processing, and mixtures to rationally adjust the naturally occurring levels of the compounds of the invention for a variety of applications.

### EXAMPLE 1: Preparation of an in vitro fermentation extract from fresh beans.

Fresh beans from *Theobroma cacao Trinitario* ICS-95 are used in this example. 500 mL of 100 mM acetic acid are used to cover the beans in autoclave bottles.

The mixture of beans/acetic acid is incubated for 35 hours at 50°C, and the solids are then concentrated by rotary evaporation at 50°C to give an "in-vitro fermentation" extract.

### EXAMPLE 2: Preparation of an in vitro fermentation extract from underfermented beans.

Underfermented beans from *Theobroma cacao Trinitario* ICS-95 are used in this example. These underfermented beans are 3-days harvested beans which have not been submitted to any treatment but have been left in an outside environment. 500 mL of 100 mM acetic acid are used to cover the beans in autoclave bottles. The mixture beans/acetic acid is incubated for 39 hours at 53°C, and the solids are then concentrated by rotary evaporation at 50°C to give an "in-vitro fermentation" extract.

### EXAMPLE 3: Preparation of a chocolate cake

We use the in-vitro fermentation extract of example 1 or example 2 in dried form. The extract is added to a cake batter, according the following formulation (percentages in weight/weight of the final product):

| | |
|---|---|
| Shortening | 11.4% |
| Chocolate liquor | 8.5% |
| In-vitro fermented extract | 0.5% |
| Flour | 22.9% |
| Sugar | 39.4% |
| Baking powder | 0.2% |
| Salt | 0.2% |
| Eggs | 16.8% |
| Aromas | 0.1% |

The batter is then baked at 150°C for 35 to 55 minutes, depending of the thickness of the cake.

### EXAMPLE 4: Preparation of a chocolate cake

We use the in-vitro fermentation extract of example 1 or example 2, in dried form. The mixture is added to a cake batter, according the following formulation (percentages in weight/weight of the final product):

| | |
|---|---|
| Shortening | 11.4% |
| Cocoa powder | 4.5% |
| In-vitro fermented extract | 4.5% |
| Flour | 22.9% |
| Sugar | 39.4% |
| Baking powder | 0.2% |
| Salt | 0.2% |
| Eggs | 16.8% |
| Aromas | 0.1 % |

The batter is then cooked for 5 to 25 minutes at 190°C depending on the thickness of the cake.

### EXAMPLE 5: Preparation of a cake with chocolate flavour

The in-vitro fermented extract of example 1 or 2 mixture is added to a cake batter, according the following formulation (percentages in weight/weight of the final product):

| | |
|---|---|
| Shortening | 16.2% |
| In-vitro fermented extract | 17% |
| Flour | 31.2% |
| Sugar | 16.6% |
| Baking powder | 0.3% |
| Salt | 0.1% |
| Eggs | 16.8% |
| Aromas | 0.1% |
| Colouring | 1.7% |

The batter is then cooked for 2 to 17 minutes at 220°C depending on the thickness of the cake.

### EXAMPLE 6: Preparation of an extruded bar with high levels of polyphenols and antioxidants

The in-vitro fermented extract of example 1 or example 2 is added to the following preparation:

| | |
|---|---|
| Glucose-fructose syrup | 20-30% |
| Fruit/Fruit preparation | 10-15% |
| Protein powder | 5-20% |
| Micronutrients | 4-5% |
| Maltodextrin | 10-15% |
| Crisp cereal/cereal | 10-33% |
| In vitro fermented extract | 5-20% |
| Fat | 2-5% |
| Flavor | 0.1-1.5% |

The ingredients are mixed until forming a homogenous mixture, under a temperature of 45°C. They are next extruded, the temperature of the extruding machine being set at 40°C. After extrusion, the bar is cooled at ambient temperature and packed.

### EXAMPLE 7: Preparation of pizza toasts

We prepare toasts with pizza-like fillings. The toasts are prepared according to the following formulation, wherein the in-vitro fermentation extract is obtained following example 1 or example 2:
1.75 kg flour
0.02 kg sugar
0.02 kg milk proteins
10 g emulsifier
0.07 g bakery powder
0.06 g shortening
1.2 kg water
0.15 kg in-vitro fermentation extract

The in-vitro fermentation extract is added as a source of antioxidants. The bread dough is knead, allowed to rest at 34°C for 45 minutes, and cooked for 50 minutes at 220°C. The bread is then cut in 15 mm slices and the pizza-like filling, according to the following preparation, is put on the top of each bread slice:
80% chopped onions
5% olive oil
2% garlic in powdered form
8% tomato puree
0.8% salt
0.1% white pepper
0.1% chilli pepper in powdered form
2% dried basil
2% in-vitro fermentation extract, as a source of antioxidants.

The proportion of filling, processed to have 70% water content, is set around 60% of the final toast weight.

### EXAMPLE 8: Preparation of a milk chocolate bar.

The in-vitro fermentation extract of example 1 or example 2 is added to the following preparation (percentages in weight by weight of the final product):

| | |
|---|---|
| Chocolate liquor | 8% |
| Milk solids | 20% |
| Sugar | 40% |
| Cocoa butter | 20% |
| Water | 1% |
| In-vitro fermented extract | 11% |

The in-vitro fermented extract is added at the beginning of the conching stage, in order to have a reaction time under heat conditions sufficient to develop the aromas of the cocoa flavor precursors in cocoa flavors. The addition of the in-vitro fermented extract allows this chocolate, poor in cocoa liquor, to have a full milk chocolate taste.

### EXAMPLE 9: Preparation of a pet food

We prepare an emulsion having the following composition, the in-vitro fermented extract being provided by following example 1 or example 2 with a further treatment to eliminate theobromine from the extract (percentages given by weight):

| | |
|---|---|
| Chopped meat | 60% |
| Cereals | 23% |
| Plant proteins | 2% |
| Water | 8% |
| In-vitro fermentation extract | 7% |

The in-vitro fermentation extract is used as a source of antioxidants and as a source of polyphenols.

### EXAMPLE 10: Preparation of a body lotion

We prepare a body lotion having the following composition, the in-vitro fermented extract being provided by the process of example 1 or example 2:

| | |
|---|---|
| Mineral oil | 8.0% |
| Isopropyl palmitate | 5.0% |
| Polyglyceryl-3-diiosostearate | 2.0% |
| Octyldodecanol | 4.0% |
| Carbomer | 0.3% |
| Cocoylglutamate sodium | 0.2% |
| Sodium hydroxide at 10% | 1.2% |
| Ascorbic acid | 0.4% |
| α-tocopherol | 0.4% |
| In-vitro fermentation extract | 0.3% |

We then complete at 100% with water. The in-vitro fermented extract is incorporated to the body lotion as a source of antioxidants and polyphenols.

## Claims

1. Low fat acetic acid extract of cocoa beans having a high level of cocoa flavors and/or flavor precursors and a high level of cocoa antioxidant compounds.

2. Low fat acetic acid extract according to claim 1, wherein the extract is an acetic acid extract from an in-vitro fermentation of cocoa beans.

3. Low fat acetic acid extract according to claim 2, wherein the cocoa beans are fresh beans or underfermented beans.

4. Low fat acetic acid extract according to one of claims 1 to 3, wherein the cocoa flavors and/or flavor precursors are in an amount of 2 to 6%, more preferably 6 to 12 %, and in a preferred embodiment 20%.

5. Low fat acetic acid extract according to one of claims 1 to 4 wherein the antioxidants are polyphenols and the polyphenol content is in an amount of 10 to 32 mg epicatechin equivalents/g extract, more preferably 30 to 100 mg epicatechin equivalents/g extract, and in a preferred embodiment 200 mg epicatechin equivalents/g extract.

6. Low fat acetic acid extract according to one of claims 1 to 4, wherein the antioxidant compounds are bioactive.

7. Cocoa flavor concentrate obtainable by using an acetic acid extract from an in-vitro fermentation of cocoa beans.

8. Cocoa flavor concentrate according to claim 7, wherein the acetic acid extract has been reacted with sugars.

9. Antioxidant concentrate obtainable by using an acetic acid extract from an in-vitro fermentation of cocoa beans.

10. Use of an acetic acid extract from an in-vitro fermentation of cocoa beans to enhance cocoa flavor.

11. Use of an acetic acid extract according to claim 10 wherein the extract is reacted with sucrose, fructose, reducing sugars and/or other sweeteners.

12. Foodstuff enriched with an acetic acid extract from an in-vitro fermentation of cocoa beans.

13. Foodstuff according to claim 12 wherein the foodstuff is chosen in the group of dietary supplement, breakfast cereals, yoghurts, dairy products, cakes, biscuits, cereal bars, coatings, drinks and beverages, sweets, powder chocolates, baby foods, gelly products, ice creams, toppings and sauces.

14. Foodstuff containing the low fat acetic extract as claimed in claim 1 and/or the cocoa flavor concentrate as claimed in claim 7 or 8, wherein the extract and/or the concentrate has been reacted with sugars.

15. Use of an in vitro fermentation acetic acid extract of cocoa beans as a source of antioxidants and cocoa flavours and/or cocoa flavour precursors.

16. Use of an in vitro fermentation extract according to claim 15, wherein the extract has been submitted to a reaction with sugars.

17. Use of an in vitro fermentation extract according to claim 9 or claim 15 in petfood, cosmetics, tire industry, iron industry, painting industry, iron industry or health products.

18. Process for obtaining an in-vitro fermentation extract comprising the steps of mixing cocoa seeds with acetic acid, heating the mixture, and concentrating the solids present in the liquid phase of the mixture.

19. Process according to claim 18 wherein the heating step is carried out at 40 to 55 DEG C, preferably 50 DEG C for 4 to 8 hours, or more preferably for 12-72 hours, in a prefered embodiment for 12 to 60 hours, in the best mode for 12 to 48 hours.

20. Process according to claim 18 or 19, wherein the heating step is carried out in autoclave bottles.

21. Process according to one of claims 18 to 20 wherein cocoa seeds are fresh seeds or underfermented seeds.

22. Process according to one of claims 18 to 21, wherein the solids concentration is carried out by rotary evaporation under vacuum.

23. Process according to one of claims 18 to 22, wherein the extract is rich in antioxidants.

## Patentansprüche

1. Fettarmer Essigsäureextrakt von Kakaobohnen mit einem hohen Anteil an Kakao-Aromastoffen und/oder -Aromavorläufern und einem hohen Anteil an Kakao-Antioxidantien-Verbindungen.

2. Fettarmer Essigsäureextrakt nach Anspruch 1, wobei der Extrakt ein Essigsäureextrakt aus einer in vitro-Fermentation von Kakaobohnen ist.

3. Fettarmer Essigsäureextrakt nach Anspruch 2, wobei die Kakaobohnen frische Bohnen oder unterfermentierte Bohnen sind.

4. Fettarmer Essigsäureextrakt nach einem der Ansprüche 1 bis 3, wobei die Kakao-Aromen und/oder -Aromavorläufer in einer Menge von 2 bis 6 %, stärker bevorzugt von 6 bis 12 % und gemäß einer bevorzugten Ausführungsform von 20 % vorliegen.

5. Fettarmer Essigsäureextrakt nach einem der Ansprüche 1 bis 4, wobei die Antioxidantien Polyphenole sind und der Polyphenolgehalt in einer Menge von 10 bis 32 mg Epicatechin-Äquivalente/g Extrakt, stärker bevorzugt von 30 bis 100 mg Epicatechin-Äquivalente/g Extrakt und in einer bevorzugten Ausführungsform von 200 mg Epicatechin-Äquivalente/g Extrakt vorliegt.

6. Fettarmer Essigsäureextrakt nach einem der Ansprüche 1 bis 4, wobei die Antioxidantien-Verbindungen bioaktiv sind.

7. Kakaoaromakonzentrat, erhältlich durch Verwendung eines Essigsäureextrakts einer in vitro-Fermentation von Kakaobohnen.

8. Kakaoaromakonzentrat nach Anspruch 7, wobei der Essigsäureextrakt mit Zuckern umgesetzt wurde.

9. Antioxidantienkonzentrat, erhältlich durch Verwendung eines Essigsäureextrakts aus einer in vitro-Fermentation von Kakaobohnen.

10. Verwendung eines Essigsäureextrakts aus einer in vitro-Fermentation von Kakaobohnen zur Verstärkung des Kakaoaromas.

11. Verwendung eines Essigsäureextrakts nach Anspruch 10, wobei der Extrakt mit Saccharose, Fructose, reduzierenden Zuckern und/oder anderen Süßungsmitteln umgesetzt wird.

12. Lebensmittel, das mit einem Essigsäureextrakt aus einer in vitro-Fermentation von Kakaobohnen angereichert ist.

13. Lebensmittel nach Anspruch 12, wobei das Lebensmittel ausgewählt ist aus der Gruppe von Nahrungsergänzungen, von Frühstückscerealien, Joghurts, Milchprodukten, Kuchen, Keksen, Cerealienriegeln, Überzügen, Drinks und Getränken, Süßigkeiten, Pulverschokoladen, Babynahrung, Geleeprodukte, Eiscremes, Toppings und Soßen.

14. Lebensmittel, das den fettarmen Essigsäureextrakt nach Anspruch 1 und/oder das Kakaoaromakonzentrat nach Anspruch 7 oder 8 enthält, wobei der Extrakt und/oder das Konzentrat mit Zuckern umgesetzt wurde.

15. Verwendung eines Essigsäureextrakts der in vitro-Fermentation von Kakaobohnen als Quelle für Antioxidantien und Kakao-Aromen und/oder Kakao-Aromavorläufer.

16. Verwendung eines in vitro-Fermentationsextrakts nach Anspruch 15, wobei der Extrakt einer Umsetzung mit Zuckern unterzogen wurde.

17. Verwendung eines in vitro-Fermentationsextrakts nach Anspruch 9 oder Anspruch 15 in einem Haustierfutter, in Kosmetika, in der Reifenindustrie, in der Eisenindustrie, in der Anstrichfarbenindustrie, in der Eisenindustrie oder in Gesundheitsprodukten.

18. Verfahren zur Gewinnung eines in vitro-Fermentationsextrakts, das die Stufen des Vermischens von Kakaosamen mit Essigsäure, das Erhitzen der Mischung und das Konzentrieren der in der flüssigen Phase der Mischung enthaltenen Feststoffe umfasst.

19. Verfahren nach Anspruch 18, wobei die Erhitzungsstufe bei 40 bis 55 °C, vorzugsweise bei 50 °C, für 4 bis 8 Stunden, oder stärker bevorzugt für 12 bis 72 Stunden, in einer bevorzugten Ausführungsform für 12 bis 60 Stunden und in der besten Ausführungsform für 12 bis 48 Stunden durchgeführt wird.

20. Verfahren nach Anspruch 18 oder 19, wobei die Erhitzungsstufe in Autoklaven-Flaschen durchgeführt wird.

21. Verfahren nach einem der Ansprüchen 18 bis 20, wobei die Kakaosamen frische Samen oder unterfermentierte Samen sind.

22. Verfahren nach einem der Ansprüche 18 bis 21, wobei die Feststoffkonzentration durch Rotationsverdampfen unter Vakuum durchgeführt wird.

23. Verfahren nach einem der Ansprüche 18 bis 22, wobei der Extrakt reich an Antioxidantien ist.

## Revendications

1. Extrait à l'acide acétique à faible teneur en matière grasse de fèves de cacao, ayant une teneur élevée en arômes et/ou précurseurs d'arômes du cacao et une teneur élevée en composés antioxydants du cacao.

2. Extrait à l'acide acétique à faible teneur en matière grasse selon la revendication 1, dans lequel l'extrait est un extrait à l'acide acétique provenant d'une fermentation de fèves de cacao *in vitro.*

3. Extrait à l'acide acétique à faible teneur en matière grasse selon la revendication 2, dans lequel les fèves de cacao sont des fèves fraîches ou des fèves sous-fermentées.

4. Extrait à l'acide acétique à faible teneur en matière grasse selon l'une quelconque des revendications 1 à 3, dans lequel les arômes et/ou précurseurs d'arômes du cacao sont présents en une quantité allant de 2 à 6%, de façon plus préférentielle de 6 à 12% et, dans un mode de réalisation préféré, égale à 20%.

5. Extrait à l'acide acétique à faible teneur en matière grasse selon l'une quelconque des revendications 1 à 4, dans lequel les antioxydants sont des polyphénols et la teneur en polyphénols est une quantité allant de 10 à 32 mg d'équivalents d'épicatéchine par gramme d'extrait, de façon plus préférentielle de 30 à 100 mg d'équivalents d'épicatéchine par gramme d'extrait et, dans un mode de réalisation préféré, égale à 200 mg d'équivalents d'épicatéchine par gramme d'extrait.

6. Extrait à l'acide acétique à faible teneur en matière grasse selon l'une quelconque des revendications 1 à 4, dans lequel les composés antioxydants sont bioactifs.

7. Concentré d'arômes de cacao, qui peut être obtenu en utilisant un extrait à l'acide acétique provenant d'une fermentation de fèves de cacao *in vitro.*

8. Concentré d'arômes de cacao selon la revendication 7, dans lequel on a fait réagir l'extrait à l'acide acétique avec des sucres.

9. Concentré d'antioxydants qui peut être obtenu en utilisant un extrait à l'acide acétique provenant d'une fermentation de fèves de cacao *in vitro.*

10. Utilisation d'un extrait à l'acide acétique provenant d'une fermentation de fèves de cacao *in vitro* pour améliorer l'arôme du cacao.

11. Utilisation d'un extrait à l'acide acétique selon la revendication 10, dans laquelle on fait réagir l'extrait avec du saccharose, du fructose, des sucres réducteurs et/ ou d'autres édulcorants.

12. Aliment enrichi en extrait à l'acide acétique provenant d'une fermentation de fèves de cacao *in vitro.*

13. Aliment selon la revendication 12, lequel aliment est choisi dans le groupe comprenant un complément diététique, des céréales pour le petit-déjeuner, des yaourts, des produits laitiers, des gâteaux, des biscuits, des barres de céréales, des nappages, des boissons et des breuvages, des sucres, des chocolats en poudre, des aliments pour bébé, des produits en gelée, des crèmes glacées, des garnitures et des sauces.

14. Aliment contenant l'extrait à l'acide acétique à faible teneur en matière grasse selon la revendication 1 et/ou le concentré d'arômes de cacao selon la revendication 7 ou la revendication 8, dans lequel on a fait réagir l'extrait et/ ou le concentré avec des sucres.

15. Utilisation d'un extrait à l'acide acétique provenant de la fermentation de fèves de cacao *in vitro* comme source d'antioxydants et d'arômes de cacao et/ou de précurseurs d'arômes de cacao.

16. Utilisation d'un extrait de fermentation *in vitro* selon la revendication 15, dans laquelle l'extrait a été soumis à une réaction avec des sucres.

17. Utilisation d'un extrait de fermentation *in vitro* selon la revendication 9 ou la revendication 15 dans des aliments pour animaux domestiques, des cosmétiques, l'industrie du pneu, l'industrie sidérurgique, l'industrie des peintures, l'industrie du fer ou les produits de santé.

18. Procédé d'obtention d'un extrait de fermentation *in vitro,* comprenant les étapes consistant à mélanger des graines de cacao avec de l'acide acétique, chauffer le mélange et concentrer les solides présents dans la phase liquide du mélange.

19. Procédé selon la revendication 18, dans lequel l'étape de chauffage se fait de 40 à 55°C, de préférence à 50°C, pendant une durée de 4 à 8 heures ou, de façon plus préférentielle, pendant 12 à 72 heures, dans un mode de réalisation préféré pendant 12 à 60 heures et dans le meilleur mode pendant 12 à 48 heures.

20. Procédé selon la revendication 18 ou 19, dans lequel l'étape de chauffage se fait dans des bouteilles autoclaves.

21. Procédé selon l'une quelconque des revendications 18 à 20, dans lequel les graines de cacao sont des graines fraîches ou des graines sous-fermentées.

22. Procédé selon l'une quelconque des revendications 18 à 21, dans lequel la concentration des solides se fait par évaporation rotative sous vide.

23. Procédé selon l'une quelconque des revendications 18 à 22, dans lequel l'extrait est riche en antioxydants.
